# EUROPÄISCHE PATENTSCHRIFT

(11) **EP 3 329 284 B1**
(45) Veröffentlichungstag und Bekanntmachung des Hinweises auf die Patenterteilung: **15.05.2019**
(21) Anmeldenummer: 17714685.9
(22) Anmeldetag: 28.03.2017
(51) Int. Cl.: G01N 33/86

(54) **VERFAHREN ZUR BESTIMMUNG VON FIBRINOGEN**
METHOD FOR DETERMINATION OF FIBRINOGEN
PROCEDE DE DETERMINATION DE FIBRINOGENES

(30) Priorität: 31.03.2016 EP 16163255
(43) Veröffentlichungstag der Anmeldung: 06.06.2018
(73) Patentinhaber: Siemens Healthcare Diagnostics Products GmbH, 35041 Marburg (DE)
(72) Erfinder: GREIS, Dirk, 35099 Burgwald (DE); ZANDER, Norbert, 35039 Marburg (DE)
(86) Internationale Anmeldenummer: PCT/EP2017/057239
(87) Internationale Veröffentlichungsnummer: WO 2017/167706

(56) Entgegenhaltungen:
- EP-A1- 2 546 637
- EP-A2- 0 699 909
- CA-A- 1 062 501
- HALBMAYER WALTER-MICHAEL ET AL: "Comparison of a new automated kinetically determined fibrinogen assay with the 3 most used fibrinogen assays (functional, derived and nephelometric) in Austrian laboratories in several clinical populations and healthy controls", HAEMOSTASIS, Bd. 25, Nr. 3, 1995, Seiten 114-123, XP009190341, ISSN: 0301-0147

## Beschreibung

Die vorliegende Erfindung liegt auf dem Gebiet der Blutgerinnungsdiagnostik und betrifft ein Verfahren zur Bestimmung der Fibrinogenkonzentration in einer humanen Plasmaprobe gemäß der Clauss-Methode.

Fibrinogen ist die wasserlösliche Vorstufe von Fibrin, welches die Matrix für den Wundverschluss bildet. Die Gerinnungsprotease Thrombin (Faktor IIa) spaltet Fibrinogen und aktiviert auf diese Weise die Fibrinbildung, also die Gerinnselbildung. Erniedrigte Fibrinogenspiegel gehen mit einer Blutungsneigung einher. Akut erhöhte Fibrinogenspiegel findet man häufig bei Entzündungen, postoperativ und in anderen Situationen. Langfristig erhöhte Fibrinogenspiegel gelten als Risikoindikator für thrombotische Erkrankungen.

Im Stand der Technik sind eine Reihe verschiedener Methoden zur Bestimmung der Fibrinogenkonzentration bekannt.

In CA 1062501 ist ein Fibrinogenbestimmungsverfahren basierend auf der Messung der Thrombinzeit beschrieben, wobei die Thrombinzeit bekanntermaßen keine präzise Bestimmung der Fibrinogenkonzentration zulässt, weil neben Fibrinogen weitere Faktoren, wie z.B. Antikoagulanzien wie Heparin oder direkte Thrombin-Inhibitoren oder auch die Gegenwart von Fibrin- oder Fibrinogen-Spaltprodukten, die Thrombinzeit beeinflussen können, und die Thrombinzeit in der Regel nur bei schweren Fibrinogen-Mangelzuständen anspricht. Bei einem Thrombinzeitverfahren wird üblicherweise eine unverdünnte Plasmaprobe mit einer verhältnismäßig geringen Thrombinmenge vermischt und die Fibrinbildung, also die Extinktionsänderung des Reaktionsansatzes photometrisch gemessen und anschließend die Gerinnungszeit bestimmt. Gemäß CA 1062501 wird jedoch keine Gerinnungszeit bestimmt, sondern es wird das Maximum der ersten Ableitung der Reaktionskurve oder mit anderen Worten die maximale Extinktionsänderung der Reaktionskurve bestimmt. Es wurde gefunden, dass die maximale Extinktionsänderung linear mit der Fibrinogenkonzentration korreliert, so dass Letztere mit Hilfe einer Kalibrationskurve, die aus einer Zuordnung von bekannten Fibrinogenkonzentrationen und maximalen Extinktionsänderungen erstellt wurde, bestimmt werden kann.

Ein wesentlich präziseres und häufig angewandtes Verfahren zur Bestimmung der Fibrinogenkonzentration ist die sogenannte Clauss-Methode (Clauss, A., Gerinnungsphysiologische Schnellmethode zur Bestimmung des Fibrinogens. 1957, Acta haemat. 17: 237-246). Der Test ist eine Variation der Thrombinzeit, bei der eine Plasmaprobe mit Thrombin vermischt wird und die Gerinnungszeit bestimmt wird. Bei der Clauss-Methode wird im Reaktionsansatz eine verhältnismäßig geringe Fibrinogenkonzentration mit einer verhältnismäßig hohen, standardisierten Thrombinkonzentration kombiniert, wodurch die Fibrinbildungsgeschwindigkeit praktisch ausschließlich mit der Fibrinogenkonzentration korreliert. Die verhältnismäßig geringe Fibrinogenkonzentration im Reaktionsansatz wird üblicherweise durch die Verwendung von vorverdünnten Plasmaproben hergestellt.

Im Reaktionsansatz wird dann die Fibrinbildung, also die Gerinnselbildung photometrisch bestimmt. Durch die Fibrinbildung trübt sich der Reaktionsansatz zunehmend, so dass durch eine Absorptionsmessung die Fibrinbildung quantitativ gemessen werden kann.

Üblicherweise wird dann die Gerinnungszeit der Probe bestimmt. Die Gerinnungszeit der Probe verhält sich proportional zur Fibrinogenmenge. Die Gerinnungszeit einer Probe ist die Zeit vom Zeitpunkt der Zugabe von Thrombin zur Probe bis zum Zeitpunkt der Messung einer fassbaren Fibrinbildung, also Trübung des Reaktionsansatzes. Dabei kann die "fassbare Fibrinbildung" als ein test- und gerätespezifischer Schwellenwert definiert sein, der -wenn er überschritten wird- die Gerinnungszeit angibt. Alternativ kann die "fassbare Fibrinbildung" ausgehend von der Signaldifferenz vor dem Start und nach Abschluss der Gerinnungsreaktion als ein test- und gerätespezifischer prozentualer Signaldifferenzwert definiert sein, der -wenn er erreicht wurde- die Gerinnungszeit angibt. Die Reaktionskinetik der Fibrinbildung in einem Clauss-Test unterscheidet sich erheblich von der Reaktionskinetik der Fibrinbildung in einem Thrombinzeit-Test. Beim Clauss-Test beginnt die Fibrinbildung je nach Fibrinogen-Konzentration bereits 3 Sekunden nach Zugabe des Thrombinreagenzes, also wesentlich früher als beim Thrombinzeit-Test, bei dem die Fibrinbildung frühestens nach 10 Sekunden einsetzt. Außerdem ist beim Clauss-Test die Fibrinbildungsgeschwindigkeit zumindest in verhältnismäßig hochkonzentrierten Proben höher als beim Thrombinzeit-Test. Die Reaktionskurven für Proben mit hoher Fibrinogenkonzentration beim Clauss-Test zeichnen sich im Vergleich zum Thrombinzeit-Test daher durch eine kurze Lag-Phase, einen steilen Anstieg und eine relativ schnell erreichte Plateauphase aus. Die Reaktionskurven für Proben mit niedriger Fibrinogenkonzentration beim Clauss-Test zeigen im Vergleich zum Thrombinzeit-Test ebenfalls eine kurze Lag-Phase, einen flachen Anstieg und eine so späte Plateauphase, dass diese meist erst nach Abschluss der Messung eintritt. Die Plateauphase ist bei der Thrombinzeit dagegen deutlich schwächer ausgeprägt oder sie entfällt sogar komplett.

Problematisch ist, dass durch die relativ hohe Verdünnung der Plasmaproben im Clauss-Test nur relativ niedrige Signalstärken erzielt werden, die durch den in optischen Systemen niedrigen Signal-Rausch-Abstand und bereits durch geringfügige Störungen, wie beispielsweise Gasbläschen im Reaktionsansatz, die durch die Verwendung gekühlter Reagenzien entstehen können, zu unpräzisen Messergebnissen führen können.

Um diese Probleme zu vermeiden, werden im Stand der Technik ungekühlte Reagenzien verwendet, und/oder es wird Kaolin als signalverstärkendes Zusatzreagenz zugegeben.

Die Verwendung ungekühlter Reagenzien hat den Nachteil, dass die Reagenzien eine verminderte Stabilität aufweisen und möglichst schnell aufgebraucht werden sollten. Außerdem sind in modernen Analysegeräten häufig ausschließlich gekühlte Aufbewahrungspositionen für Reagenzbehälter vorgesehen, so dass ohne Weiteres gar kein ungekühltes Reagenz vorgesehen werden kann. Die Verwendung signalverstärkender Zusatzreagenzien ist ebenfalls unerwünscht, einerseits aus wirtschaftlichen Gründen und andererseits, weil sich durch den zusätzlich erforderlichen Pipettierschritt die Abarbeitung des Tests verlängert. Darüber hinaus kann sich das bildende Gerinnsel besonders bei niedrigen Plasmaspiegeln von Fibrinogen zusammenballen und als "Fibrin-Kaolin-Blase" im Reaktionsansatz herumschwimmen.

Ferner ist es problematisch, dass bei der Bestimmung der Gerinnungszeit einer Probe im Clauss-Test idealerweise mit der Messung unmittelbar nach der Zugabe des Thrombins zur Probe begonnen werden sollte. In der Praxis vergeht jedoch bei automatisierter Durchführung auf Analysengeräten eine kurze Zeitspanne (mehrere Sekunden) zwischen der Zugabe des Thrombinreagenzes in das Reaktionsgefäß und dem Absetzen des Reaktionsgefäßes in die Messposition. Bei sehr hohen Plasmaspiegeln von Fibrinogen kann es aber während dieser kurzen Zeit bereits zu einem Start der Reaktion und damit zu einem Signalanstieg kommen. In diesem Fall ist eine klassische Bestimmung der Gerinnungszeit schwierig, da für die "fassbare Fibrinbildung", sowohl über einen test- und gerätespezifischen Schwellenwert oder alternativ über die prozentuale Signaldifferenz bezogen auf Start und Abschluss der Gerinnungsreaktion, der Startwert bekannt sein muss.

Die der Erfindung zugrunde liegenden Aufgabe besteht also darin, die Clauss-Methode zur Bestimmung der Fibrinogenkonzentration in einer humanen Plasmaprobe so zu modifizieren, dass eine automatische Abarbeitung mit hoher Präzision durchführbar ist und dass auf die Verwendung ungekühlter Reagenzien und den Einsatz signalverstärkender Zusatzreagenzien, wie beispielsweise Kaolin, verzichtet werden kann.

Die Aufgabe wird dadurch gelöst, dass nicht mehr die bisher gebräuchliche Gerinnungszeit der Probe bestimmt wird, sondern dass die Reaktionskinetik der Fibrinbildung gemessen wird und die Reaktionsgeschwindigkeit zur Fibrinogenkonzentrationsbestimmung verwendet wird.

Gegenstand der vorliegenden Anmeldung ist also ein Verfahren zur Bestimmung der Fibrinogenkonzentration in einer humanen Plasmaprobe gemäß der Clauss-Methode, das Verfahren umfassend die Schritte:
- Bereitstellen eines Reaktionsansatzes enthaltend die Plasmaprobe und Thrombin,
- Messen der Extinktionswerte des Reaktionssatzes über die Zeit,
- Erstellen einer Reaktionskurve aus den gemessenen Extinktionswerten über die Zeit,
- Ermitteln der maximalen Extinktionsänderung mittels eines Regressionsverfahrens und
- Bestimmen der Fibrinogenkonzentration mit Hilfe einer Kalibrationskurve, die aus einer Zuordnung von bekannten Fibrinogenkonzentrationen und maximalen Extinktionsänderungen erstellt wurde.

Das Mischungsverhältnis von Plasmaprobe und Thrombineinheiten zur Bereitstellung eines Reaktionsansatzes gemäß der Clauss-Methode, d.h. die Kombination einer verhältnismäßig geringen Fibrinogenkonzentration (aus der Probe) mit einer verhältnismäßig hohen Thrombinkonzentration, so dass die Fibrinbildungsgeschwindigkeit praktisch ausschließlich mit der Fibrinogenkonzentration korreliert, ist dem Fachmann hinreichend bekannt (siehe auch Thomas, L., Labor und Diagnose, 7. Auflage, TH-Books Verlagsgesellschaft mbH, Frankfurt/Main, 2008, Kapitel 16.15.2).

Vorzugsweise enthält der Reaktionsansatz dazu etwa 0,03 bis 0,4 mg/mL Fibrinogen und etwa 25 bis 40 IU/mL Thrombin. Diese Konzentrationen können beispielsweise dadurch erhalten werden, dass eine ca. 1:10 Verdünnung einer zu untersuchenden Plasmaprobe in einem Puffer und ein gepuffertes Thrombinreagenz mit einer Konzentration von ca. 100 IU/mL Thrombin in einem Verhältnis von 2+1 gemischt werden. Damit können Fibrinogenkonzentrationen von etwa 0,5 g/L bis 6,0 g/L bestimmt werden. Durch Änderung der Vorverdünnung der Probe kann der gesamte physiologisch vorkommende Bereich von Fibrinogen im menschlichen Plasma gemessen werden.

Das Messen der Extinktionswerte des Reaktionssatzes über die Zeit kann photometrisch, d.h. durch die Messung der Lichtabschwächung eines durch den Reaktionsansatz gesendeten Lichtstrahls, oder nephelometrisch, d.h. durch die Messung von Streulichtanteilen eines durch den Reaktionsansatz gesendeten Lichtstrahls, erfolgen. Idealerweise wird mit der Messung unmittelbar nach der Zugabe des Thrombins zur Probe begonnen, und die Messung der Extinktionswerte wird kontinuierlich bis zum Abschluss der Fibrinbildung durchgeführt. In der Praxis vergeht jedoch bei automatisierter Durchführung auf Analysengeräten eine kurze Zeitspanne (mehrere Sekunden) zwischen der Zugabe des Thrombinreagenzes in das Reaktionsgefäß mit anschließendem Mischen und dem Absetzen des Reaktionsgefäßes in die Messposition.

Es wurde gefunden, dass sich das erfindungsgemäße Verfahren insbesondere eignet für Testprotokolle, bei denen eine sofortige Messung der Extinktionswerte nicht möglich ist, beispielsweise weil der Reaktionsansatz erst in die Messstation transportiert werden muss. In einer spezifischen Ausführungsform, bei der das Bereitstellen eines Reaktionsansatzes die Zugabe von Thrombin zur Plasmaprobe umfasst, liegt zwischen dem Zeitpunkt der Thrombinzugabe zur Plasmaprobe und dem Beginn des Messens der Extinktionswerte des Reaktionssatzes eine Zeitspanne von 0,5 bis 5 Sekunden, bevorzugt von 3 bis 4 Sekunden.

Das zur Ermittlung der maximalen Extinktionsänderung angewandte Regressionsverfahren ist eine kinetische Auswertemethode, die dem Fachmann bekannt ist und bei der in Abhängigkeit von einer in einem ersten Auswertungsschritt grob ermittelten Steigung der Reaktionskurve in einem zweiten Auswertungsschritt ein individuelles und optimiertes Regressionsintervall für die Berechnung der maximalen Reaktionsgeschwindigkeit, also der maximalen Extinktionsänderung, bestimmt wird.

Vorzugsweise umfasst das Regressionsverfahren die folgenden Schritte:
- einen ersten Schritt, in dem mit einem vorab festgelegten ersten Regressionsintervall eine erste, vorläufige maximale Extinktionsänderung in der Reaktionskurve bestimmt wird,
- einen zweiten Schritt, in dem anhand der bestimmten ersten, vorläufigen maximalen Extinktionsänderung und anhand von vorab festgelegten Parametern ein zweites Regressionsintervall bestimmt wird, und
- einen dritten Schritt, in dem mit dem zweiten Regressionsintervall die maximale Extinktionsänderung bestimmt wird.

Vorzugsweise wird also zunächst innerhalb eines test- und gerätespezifischen definierten Auswertebereichs beginnend ab dem ersten Messwert (der so nahe wie möglich am Zeitpunkt der Thrombinzugabe liegt) über ein test- und gerätespezifisches Regressionsintervall die Steigung (= Reaktionsgeschwindigkeit bzw. Extinktionsänderung) per Regression nach der Methode der kleinsten Quadrate berechnet. Diese Berechnung wird mehrmals wiederholt, indem der zweite, dritte, vierte usw. Messwert als Startpunkt des Regressionsintervalls verwendet wird.

Die so ermittelte vorläufige maximale Steigung (Grobsteigung) dient zur Berechnung des endgültigen Regressionsintervalls über eine Potenzfunktion mit empirisch, vorab festgelegten Parametern Faktor und Exponent. Über einen negativen Exponenten wird die gewünschte Wirkung erzielt, dass mit wachsender Steigung das Regressionsintervall kürzer wird. Grundsätzlich kann die Berechnung des endgültigen Regressionsintervalls jedoch auch über eine andere Gleichung erfolgen oder einer in der Software hinterlegten Tabelle entnommen werden.

Mit dem berechneten, endgültigen Regressionsintervall wird die Ermittlung der endgültigen maximalen Steigung wie bereits oben beschrieben durchgeführt, wiederum beginnend ab dem ersten Messwert per Regression nach der Methode der kleinsten Quadrate. Diese Berechnung wird mehrmals wiederholt, indem der zweite, dritte, vierte usw. Messwert als Startpunkt des Regressionsintervalls verwendet wird.

Es wurde gefunden, dass mit der Anwendung dieses zweistufigen Regressionsverfahrens zur Ermittlung der maximalen Steigung einer Clauss-Test-Reaktionskinetik die Fibrinogenkonzentration sowohl in Proben mit hoher als auch mit niedriger Fibrinogenkonzentration präzise bestimmt werden kann, auch wenn Störungen in der Reaktionskinetik vorliegen, wie beispielsweise Gasbläschen im Reaktionsansatz, die das eigentliche Messsignal überlagern und damit eine "falsche" maximale Steigung enthalten.

Die Bestimmung der Fibrinogenkonzentration der Probe erfolgt nun mit Hilfe einer Kalibrationskurve, die aus einer Zuordnung von bekannten Fibrinogenkonzentrationen und maximaler Steigung (= maximale Extinktionsänderung) erstellt wurde.

Ein weiterer Gegenstand der vorliegenden Erfindung ist ein automatisches Analysegerät mit mindestens einer Pipettiervorrichtung, einer photometrischen oder nephelometrischen Messstation und einer Datenverabeitungseinheit, dadurch gekennzeichnet, dass das Analysegerät ferner einer Steuereinrichtung umfasst, die so konfiguriert ist, dass sie ein Verfahren zur Bestimmung der Fibrinogenkonzentration gemäß der Clauss-Methode mit den folgenden Schritten steuert:
- Bereitstellen eines Reaktionsansatzes enthaltend eine Plasmaprobe und Thrombin,
- Messen der Extinktionswerte des Reaktionssatzes über die Zeit,
- Erstellen einer Reaktionskurve aus den gemessenen Extinktionswerten über die Zeit,
- Ermitteln der maximalen Extinktionsänderung mittels eines Regressionsverfahrens und
- Bestimmen der Fibrinogenkonzentration mit Hilfe einer Kalibrationskurve, die aus einer Zuordnung von bekannten Fibrinogenkonzentrationen und maximalen Extinktionsänderungen erstellt wurde.

Derartige automatische Analysegeräte werden routinemäßig in der Analytik, der Forensik, der Mikrobiologie und der klinischen Diagnostik verwendet, um eine Vielzahl von Proben in einer Vielzahl von Untersuchungsmethoden schnell, exakt und reproduzierbar zu analysieren.

Ein erfindungsgemäßes Analysegerät mit einer Steuereinrichtung, die so konfiguriert ist, dass sie die Durchführung eines Clauss-Tests in Kombination mit der oben beschriebenen kinetischen Auswertung steuert, hat den Vorteil, dass es die Durchführung einer präzisen Fibrinogenbestimmung ermöglicht, wobei für die Reagenzien, die für die Bereitstellung des Reaktionsansatzes verwendet werden, insbesondere für das Thrombinreagenz, keine ungekühlten Aufbewahrungspositionen vorgesehen sein müssen.

Das Bereitstellen eines Reaktionsansatzes enthaltend die Plasmaprobe und Thrombin in dem automatischen Analysegerät wird typischerweise mit Hilfe von einer oder mehreren automatischen Pipettiervorrichtungen durchgeführt.

Die Messung der Extinktionswerte des Reaktionssatzes über die Zeit erfolgt typischerweise in der photometrischen oder nephelometrischen Messstation.

Unter einer "photometrischen Messstation" ist eine Messeinheit zu verstehen, die mindestens eine Lichtquelle und mindestens einen Lichtdetektor aufweist und die so ausgestaltet ist, dass sie die Messung der Extinktion von Licht einer bestimmten Wellenlänge in einer Probe ermöglicht. Typischerweise ist die Wellenlänge des von der Lichtquelle emittierten Lichts so gewählt, dass es von einer in der Probe nachzuweisenden Substanz, hier Fibrin, abgeschwächt (absorbiert, reflektiert oder gestreut) wird.

Unter einer "nephelometrischen Messstation" ist eine Messeinheit zu verstehen, die mindestens eine Lichtquelle und mindestens einen Lichtdetektor aufweist und die so ausgestaltet ist, dass sie die Messung der Extinktion von Licht in einer Probe ermöglicht. Typischerweise ist die Anordnung von Lichtquelle und Lichtdetektor so gewählt, dass das Streulicht messbar ist, das von in der Probe nachzuweisenden Makromolekülen, beispielsweise von Partikelaggregaten, die infolge einer Analyt-abhängigen Reaktion in einem Reaktionsansatz entstehen, oder hier von Fibrin gestreut wird.

Für die Bestimmung der Fibrinogenkonzentration mit Hilfe einer Kalibrationskurve, ist die entsprechende Zuordnung von bekannten Fibrinogenkonzentrationen und maximalen Extinktionsänderungen, vorzugsweise in der Datenverabeitungseinheit gespeichert. In der Datenverarbeitungseinheit erfolgt dann auch das Ermitteln der Fibrinogenkonzentration durch Ablesen der der ermittelten maximalen Extinktionsänderung des Reaktionsansatzes entsprechenden Fibrinogenkonzentration.

Eine Ausführungsform eines erfindungsgemäßen automatischen Analysegeräts umfasst ferner eine erste Aufnahmeposition für Reaktionsgefäße, die für die Bereitstellung von Reaktionsansätzen vorgesehen ist, und eine zweite Aufnahmeposition für Reaktionsgefäße, die der Messstation zugeordnet ist, und eine Vorrichtung zum Transport eines Reaktionsgefäßes von der ersten in die zweite Aufnahmeposition, wobei die Steuereinrichtung ferner so konfiguriert ist, dass sie ferner folgende Schritte steuert:
- in der ersten Aufnahmeposition für Reaktionsgefäße Bereitstellen eines Reaktionsgefäßes enthaltend den Reaktionsansatz durch Zugabe von Thrombin zu einer in dem Reaktionsgefäß befindlichen Plasmaprobe,
- Transport des Reaktionsgefäßes von der ersten in die zweite Aufnahmposition und dann
- Messen der Extinktionswerte des Reaktionssatzes über die Zeit,
wobei zwischen dem Zeitpunkt der Thrombinzugabe zur Plasmaprobe und dem Beginn des Messens der Extinktionswerte des Reaktionssatzes eine Zeitspanne von 0,5 bis 5 Sekunden, bevorzugt von 3 bis 4 Sekunden liegt.

Bei dem Reaktionsgefäß handelt es sich vorzugsweise um eine transparente, röhrchenförmige Küvette aus Kunststoff oder Glas.

Unter einer "Aufnahmeposition" für ein Reaktionsgefäß ist ein Ort gemeint, der für die Platzierung eines Reaktionsgefäßes vorgesehen ist. Dabei handelt es sich häufig um eine baulich angepasste Aufnahmevorrichtung, die eine standsichere Aufbewahrung des Flüssigkeitsbehälters ermöglicht, wie beispielsweise Hülsen, in die ein spezifisch ausgestaltetes Reaktionsgefäß formschlüssig eingeführt werden kann. Eine Aufnahmeposition befindet sich häufig an beweglichen Aufnahmeeinrichtungen, wie beispielsweise drehbaren Küvettentellern oder -ringen, die eine Vielzahl von Aufnahmepositionen aufweisen. Aufnahmepositionen, die einer Messstation zugeordnet sind, sind so ausgestaltet und angeordnet, dass in ihnen die Messung eines in einem Reaktionsgefäß befindlichen Reaktionssatzes möglich ist.

Für den Transport von Reaktionsgefäßen zwischen zwei Aufnahmepositionen, die sich auf räumlich getrennten Aufnahmeeinrichtungen befinden, z.B. zwischen einer ersten Aufnahmeposition, auf die eine Pipettiervorrichtung Zugriff hat und in der Reaktionsansätze bereitgestellt und inkubiert werden können und einer zweiten Aufnahmeposition, in der die Messung des Reaktionsätze stattfindet, sind häufig Greifer vorgesehen, die an einem horizontal und vertikal bewegbaren Transferarm befestigt sind.

Die Erfindung wird im Folgenden anhand einer Zeichnung erläutert.

Darin zeigen
- FIG. 1: ein erfindungsgemäßes automatisches Analysegerät;
- FIG. 2: eine Reaktionskinetik einer Plasmaprobe mit hoher Fibrinogenkonzentration in der Fibrinogenmethode nach Clauss;
- FIG. 3: eine Reaktionskinetik einer Plasmaprobe mit niedriger Fibrinogenkonzentration in der Fibrinogenmethode nach Clauss;
- FIG. 4: eine erfindunsgemäße Kalibrationskurve für die Fibrinogenmethode nach Clauss;
- FIG. 5 und 6: Kalibrationskurven gemäß dem Stand der Technik mit Gerinnungszeiten für die Fibrinogenmethode nach Clauss.

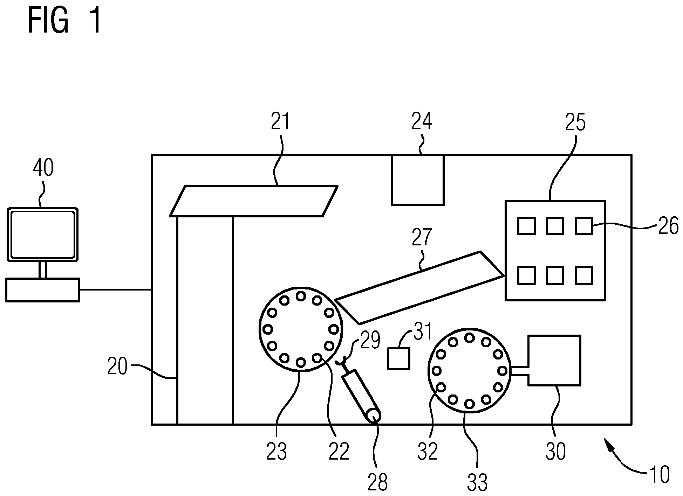

FIG. 1 ist eine schematische Darstellung eines automatischen Analysegeräts 10 mit einigen darin enthaltenen Bauteilen.

Hierbei werden nur die wichtigsten Bauteile stark vereinfacht dargestellt, um die grundsätzliche Funktion des automatischen Analysegeräts 10 zu erläutern, ohne hierbei detailliert die einzelnen Teile jedes Bauteils darzustellen.

Das automatische Analysegerät 10 ist dafür ausgebildet, vollautomatisch verschiedenste Analysen von Blut oder anderen Körperflüssigkeiten durchzuführen, ohne dass hierfür Aktivitäten eines Benutzers notwendig wären. Notwendige Eingriffe eines Benutzers beschränken sich vielmehr auf Wartung oder Reparatur und Nachfüllarbeiten, wenn z.B. Küvetten nachgefüllt oder Flüssigkeitsbehälter ausgetauscht werden müssen.

Die Patientenproben werden dem automatischen Analysegerät 10 in Primärprobengefäßen auf nicht näher dargestellten Schlitten über eine Zuführungsschiene 20 zugeführt. Informationen hinsichtlich der pro Probe durchzuführenden Analysen können beispielsweise mittels auf den Probengefäßen angebrachten Strichcodes übergeben werden, die in dem automatischen Analysegerät 10 ausgelesen werden. Aus den Probengefäßen werden mit Hilfe einer ersten Pipettiervorrichtung 21 Probenaliquots mittels einer Pipettiernadel entnommen.

Die Probenaliquots werden ebenfalls nicht näher dargestellten Küvetten zugeführt, die in Aufnahmepositionen 22 einer drehbaren, auf 37 °C temperierten Inkubationseinrichtung 23 angeordnet sind. Die Küvetten werden aus einem Küvettenvorratsbehälter 24 entnommen. In dem auf ca. 8-10 °C gekühlten Reagenzgefäßvorratsbehälter 25 werden Reagenzgefäße 26 mit verschiedenen Reagenzflüssigkeiten, wie z.B. mit einer Thrombinreagenzflüssigkeit, aufbewahrt. Reagenzflüssigkeit wird mittels der Pipettiernadel einer zweiten Pipettiervorrichtung 27 aus einem Reagenzgefäß 26 entnommen und zur Bereitstellung eines Reaktionsansatzes in eine Küvette, die bereits ein Probenaliquot, beispielsweise eine Plasmaprobe, enthält, abgegeben. Die Küvette mit dem Reaktionsansatz wird von dem Transferarm 28 mit einem Klemmgreifer 29 aus einer Aufnahmeposition 22 der Inkubationseinrichtung 23 entnommen und zum Durchmischen des Reaktionsansatzes zu einer Schütteleinrichtung 31 transferiert. Nach Beendigung des Mischvorgangs wird die Küvette weiter in eine Aufnahmeposition 32 der drehbaren Aufnahmevorrichtung 33 für die photometrische Messstation 30 transportiert, wo die Extinktion des Reaktionsansatzes gemessen wird.

Der gesamte Prozess wird von einer zentralen Steuereinheit 40, wie z.B. von einem über eine Datenleitung angeschlossenen Rechner gesteuert, unterstützt durch eine Vielzahl weiterer, nicht näher dargestellter elektronischer Schaltungen und Mikroprozessoren innerhalb des automatischen Analysegeräts 10 und seiner Bauteile.

Das nachfolgende Beispiel ist zur Veranschaulichung der Erfindung nicht aber als Einschränkung anzusehen.

### BEISPIEL

### Durchführung der Fibrinogenmethode nach Clauss

Es wurden 1:12,5-Verdünnungen von humanen Plasmaproben im Fibrinogenkonzentrationsbereich von ca. 1 g/L bis ca. 5 g/L in Owren's Veronal Puffer hergestellt. 100 µL dieser verdünnten Proben wurden bei +37 °C temperiert und 50 µL temperiertes Thrombinreagenz (ca. 100 IU/mL) wurden zugefügt. Die Reaktionsansätze wurden gemischt, und die Extinktion wurde photometrisch mit Licht einer Wellenlänge von 405 nm über einen Zeitraum von etwa 80 Sekunden gemessen. Die Bereitstellung der Reaktionsansätze sowie die Messung der Extinktion wurden in einem automatischen Analysegerät durchgeführt.

### Erfindungsgemäße Auswertung der Reaktionskinetiken

Die Auswertung der Reaktionskinetiken, also der über die Zeit gemessenen Extinktionswerte, erfolgte erfindungsgemäß, indem die maximale Extinktionsänderung einer Reaktionskinetik mittels eines Regressionsverfahrens ermittelt wurde.

Auf dieselbe Art wurde eine Kalibrationskurve mit Plasmaproben bekannter Fibrinogenkonzentrationen und den dazugehörigen, erfindungsgemäß ermittelten maximalen Extinktionsänderungen erstellt.

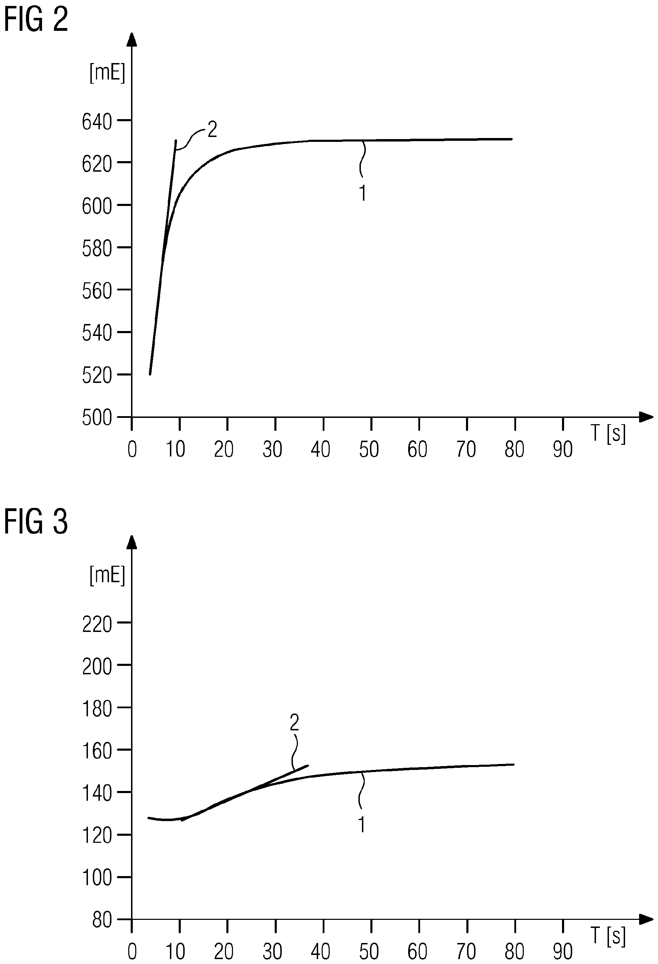

FIG. 2 zeigt den zeitlichen Verlauf der Extinktionsmesswerte (mE), also die Reaktionskinetik (1), einer Plasmaprobe mit einer hohen Fibrinogenkonzentration (ca. 5 g/L). Die Reaktion, also die Fibrinbildung, hat bereits beim Einsetzen des Reaktionsgefäßes in den Messkanal (ca. 3-4 Sekunden nach Reagenzzugabe) begonnen. Hier kann eine klassische Bestimmung der Gerinnungszeit mittels der Signaldifferenz-Methode schon nicht mehr exakt durchgeführt werden, da das Startsignal unbekannt ist. Die erfindungsgemäße Bestimmung der maximalen Reaktionsgeschwindigkeit mittels eines angepassten Regressionsintervalls ist hier die Methode der Wahl. Das angepasste Regressionsintervall ist hier sehr kurz (2 Sekunden). Die Gerade (2) ist die Gerade der maximalen Reaktionsgeschwindigkeit (Extinktionsänderung).

FIG. 3 zeigt den zeitlichen Verlauf der Extinktionsmesswerte (mE), also die Reaktionskinetik (1), einer Plasmaprobe mit einer niedrigen Fibrinogenkonzentration (ca. 1 g/L). Hier geschieht die Umwandlung von Fibrinogen in Fibrin sehr langsam und teilweise ohne klar ausgeprägtes Endplateau. In diesem Fall ist ebenfalls die Bestimmung der Reaktionsgeschwindigkeit die Methode der Wahl. Das angepasste Regressionsintervall ist hier verlängert (9 Sekunden). Die Gerade (2) ist die Gerade der maximalen Reaktionsgeschwindigkeit (Extinktionsänderung).

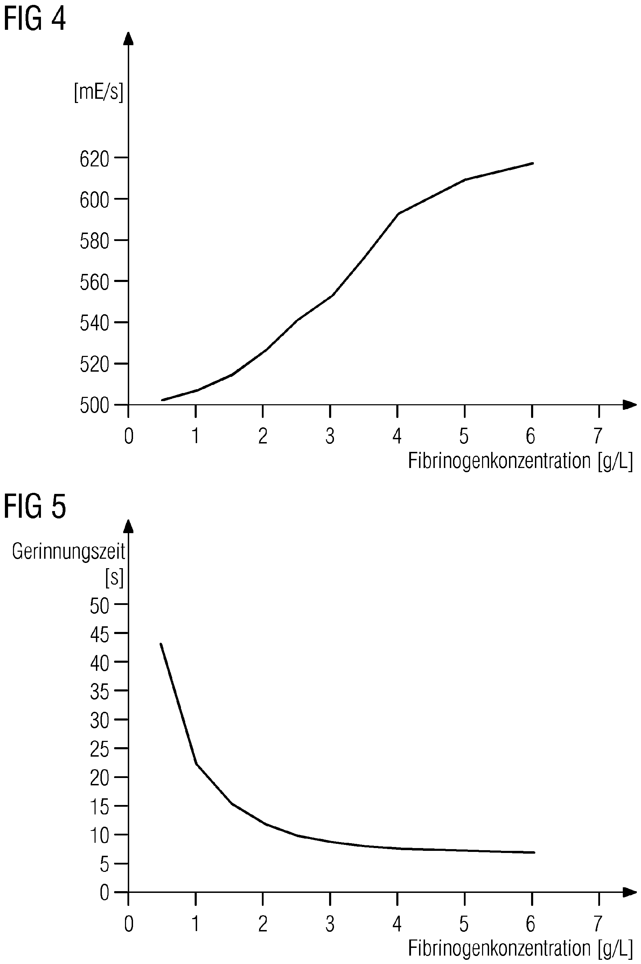

FIG. 4 zeigt eine Kalibrationskurve für die erfindungsgemäße Bestimmung der Fibrinogenkonzentration. Es wurden 10 Plasmaproben bekannter Fibrinogenkonzentration zwischen 0,5 und 6,0 g/L mit dem erfindungsgemäßen Verfahren analysiert und jeweils die maximale Extinktionsänderung [mE/s] bestimmt. Es ergibt sich in allen Intervallen eine gute Spreizung zwischen den Stützpunkten. Durch eine Änderung der Probenverdünnung, z.B. als 1:4,2-Verdünnung von Proben mit einer Fibrinogenkonzentration von weniger als 1,0 g/L oder einer 1:25-Verdünnung von Proben mit einer Fibrinogenkonzentration von mehr als 6,0 g/L ließe sich der messbare Fibrinogenkonzentrationsbereich entsprechend erweitern.

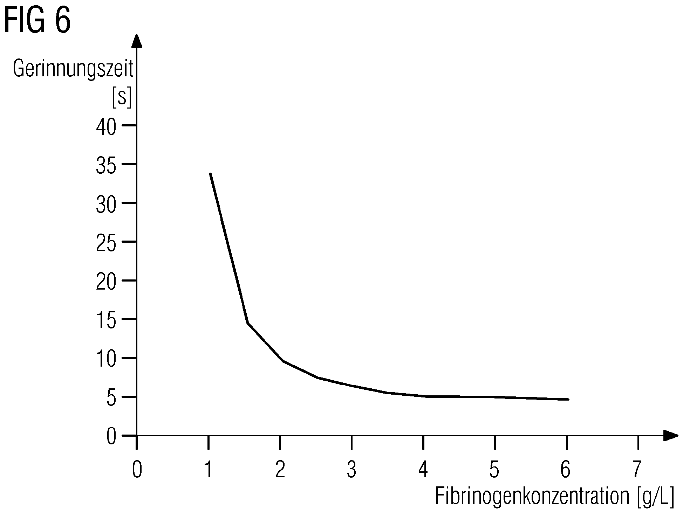

Zum Vergleich sind in den FIG. 5 und 6 Kalibrationskurven für die bisher üblichen Fibrinogenbestimmungsmethoden nach Clauss mit einer Auswertung der Gerinnungszeit über Schwellenwerte (FIG. 5) oder über die Signaldifferenz in Prozent (FIG. 6) gezeigt. In beiden Fällen ist der Verlauf der Kalibrationskurve oberhalb von etwa 4 g/L bereits so flach, dass eine sinnvolle Auswertung der Rohwerte praktisch nicht mehr möglich ist. Proben mit einer Fibrinogenkonzentration von mehr als 4 g/L müssten also bereits stärker verdünnt und nochmals gemessen werden. Der Grund für die starke Abflachung liegt unter anderem in der bereits gestarteten Reaktion zum Zeitpunkt der ersten Messwertaufnahme.

Darüber hinaus ist eine Auswertung von mit der Schwellenwert-Methode bestimmten Gerinnungszeiten bei einer Konzentration von 0,5 g/L nicht möglich, da das Signal die Mindestschwelle nicht erreicht. Eine Reduktion des Schwellenwertes als mögliche Lösung bringt die Gefahr von Falschauswertungen auf Grund von Überlagerungen in der Reaktionskurve (z.B. durch Luftblasen) mit sich.

### BEZUGSZEICHENLISTE

- 1: Reaktionskinetik
- 2: Gerade

- 10: Analysegerät
- 20: Zuführungsschiene
- 21: Pipettiervorrichtung
- 22: Aufnahmeposition
- 23: Inkubationseinrichtung
- 24: Küvettenvorratsbehälter
- 25: Reagenzgefäßvorratsbehälter
- 26: Reagenzgefäß
- 27: Pipettiervorrichtung
- 28: Transferarm
- 29: Klemmgreifer
- 30: Messstation
- 31: Schütteleinrichtung
- 32: Aufnahmeposition
- 33: Aufnahmevorrichtung
- 40: Steuereinheit

## Patentansprüche

1. Verfahren zur Bestimmung der Fibrinogenkonzentration in einer Plasmaprobe gemäß der Clauss-Methode, das Verfahren umfassend die Schritte:
• Bereitstellen eines Reaktionsansatzes enthaltend die Plasmaprobe und Thrombin,
• Messen der Extinktionswerte des Reaktionssatzes über die Zeit,
• Erstellen einer Reaktionskurve aus den gemessenen Extinktionswerten über die Zeit,
• Ermitteln der maximalen Extinktionsänderung mittels eines Regressionsverfahrens und
• Bestimmen der Fibrinogenkonzentration mit Hilfe einer Kalibrationskurve, die aus einer Zuordnung von bekannten Fibrinogenkonzentrationen und maximalen Extinktionsänderungen erstellt wurde.

2. Verfahren gemäß Anspruch 1, wobei Regressionsverfahren die folgenden Schritte umfasst:
• einen ersten Schritt, in dem mit einem vorab festgelegten ersten Regressionsintervall eine erste, vorläufige maximale Extinktionsänderung in der Reaktionskurve bestimmt wird,
• einen zweiten Schritt, in dem anhand der bestimmten ersten, vorläufigen maximalen Extinktionsänderung und anhand von vorab festgelegten Parametern ein zweites Regressionsintervall bestimmt wird, und
• einen dritten Schritt, in dem mit dem zweiten Regressionsintervall die maximale Extinktionsänderung bestimmt wird.

3. Verfahren gemäß Anspruch 1, wobei das Bereitstellen eines Reaktionsansatzes enthaltend die Plasmaprobe und Thrombin, die Zugabe von Thrombin zur Plasmaprobe umfasst und wobei zwischen dem Zeitpunkt der Thrombinzugabe zur Plasmaprobe und dem Beginn des Messens der Extinktionswerte des Reaktionssatzes eine Zeitspanne von 0,5 bis 5 Sekunden, bevorzugt von 3 bis 4 Sekunden liegt.

4. Automatisches Analysegerät (10) mit mindestens einer Pipettiervorrichtung (21, 27), einer photometrischen oder nephelometrischen Messstation (30) und einer Datenverarbeitungseinheit, **dadurch gekennzeichnet, dass** das Analysegerät (10) ferner einer Steuereinrichtung umfasst, die so konfiguriert ist, dass sie ein Verfahren zur Bestimmung der Fibrinogenkonzentration in einer Plasmaprobe gemäß der Clauss-Methode steuert, wobei das Verfahrendie folgenden Schritte umfasst:
• Bereitstellen eines Reaktionsansatzes enthaltend die Plasmaprobe und Thrombin,
• Messen der Extinktionswerte des Reaktionssatzes über die Zeit,
• Erstellen einer Reaktionskurve aus den gemessenen Extinktionswerten über die Zeit,
• Ermitteln der maximalen Extinktionsänderung mittels eines Regressionsverfahrens und
• Bestimmen der Fibrinogenkonzentration mit Hilfe einer Kalibrationskurve, die aus einer Zuordnung von bekannten Fibrinogenkonzentrationen und maximalen Extinktionsänderungen erstellt wurde.

5. Automatisches Analysegerät (10) gemäß Anspruch 4, ferner umfassend eine erste Aufnahmeposition (22) für Reaktionsgefäße, die für die Bereitstellung von Reaktionsansätzen vorgesehen ist, und eine zweite Aufnahmeposition (32) für Reaktionsgefäße, die der Messstation (30) zugeordnet ist, und eine Vorrichtung (10, 11) zum Transport eines Reaktionsgefäßes von der ersten in die zweite Aufnahmeposition, wobei die Steuereinrichtung ferner so konfiguriert ist, dass sie ferner folgende Schritte steuert:
• in der ersten Aufnahmeposition (22) Bereitstellen eines Reaktionsgefäßes enthaltend den Reaktionsansatz durch Zugabe von Thrombin zu einer in dem Reaktionsgefäß befindlichen Plasmaprobe,
• Transport des Reaktionsgefäßes von der ersten in die zweite Aufnahmposition (23) und dann
• Messen der Extinktionswerte des Reaktionssatzes über die Zeit in der Messstation (30),
wobei zwischen dem Zeitpunkt der Thrombinzugabe zur Plasmaprobe und dem Beginn des Messens der Extinktionswerte des Reaktionssatzes eine Zeitspanne von 0,5 bis 5 Sekunden, bevorzugt von 3 bis 4 Sekunden liegt.

## Claims

1. Method for determining the concentration of fibrinogen in a plasma sample according to the Clauss method, the method comprising the steps:
• providing a reaction mix containing the plasma sample and thrombin,
• measuring the absorbance values of the reaction mixture over time,
• preparing a reaction curve from the measured absorbance values over time,
• ascertaining the maximum absorbance change by means of a regression method and
• determining the concentration of fibrinogen with the aid of a calibration curve which has been prepared from an assignment of known fibrinogen concentrations and maximum absorbance changes.

2. Method according to Claim 1, wherein regression method comprises the following steps:
• a first step in which a first, provisional maximum absorbance change in the reaction curve is determined using a predetermined first regression interval,
• a second step in which a second regression interval is determined on the basis of the first, provisional maximum absorbance change that is determined and on the basis of predetermined parameters, and
• a third step in which the maximum absorbance change is determined using the second regression interval.

3. Method according to Claim 1, wherein the provision of a reaction mix containing the plasma sample and thrombin comprises the addition of thrombin to the plasma sample and wherein there is a period of time from 0.5 to 5 seconds, preferably from 3 to 4 seconds, between the time of addition of thrombin to the plasma sample and the start of the measurement of the absorbance values of the reaction mixture.

4. Automatic analyzer (10) comprising at least one pipetting device (21, 27), a photometric or nephelometric measurement station (30) and a data processing unit, **characterized in that** the analyzer (10) further comprises a controller configured in such a way that it controls a method for determining the concentration of fibrinogen in a plasma sample according to the Clauss method, the method comprising the following steps:
• providing a reaction mix containing the plasma sample and thrombin,
• measuring the absorbance values of the reaction mixture over time,
• preparing a reaction curve from the measured absorbance values over time,
• ascertaining the maximum absorbance change by means of a regression method and
• determining the concentration of fibrinogen with the aid of a calibration curve which has been prepared from an assignment of known fibrinogen concentrations and maximum absorbance changes.

5. Automatic analyzer (10) according to Claim 4, further comprising a first accommodation position (22) for reaction vessels, which position is intended for the provision of reaction mixes, and a second accommodation position (32) for reaction vessels, which position is assigned to the measurement station (30), and a device (10, 11) for transporting a reaction vessel from the first accommodation position to the second, wherein the controller is further configured in such a way that it further controls the following steps:
• providing in the first accommodation position (22) a reaction vessel containing the reaction mix by addition of thrombin to a plasma sample present in the reaction vessel,
• transporting the reaction vessel from the first accommodation position to the second (23), and then
• measuring the absorbance values of the reaction mixture over time in the measurement station (30),
wherein there is a period of time from 0.5 to 5 seconds, preferably from 3 to 4 seconds, between the time of addition of thrombin to the plasma sample and the start of the measurement of the absorbance values of the reaction mixture.

## Revendications

1. Procédé de détermination de la concentration en fibrinogène d'un échantillon de plasma suivant la méthode de Clauss, le procédé comprenant les stades :
• on se procure une masse réactionnelle contenant l'échantillon de plasma et de la thrombine,
• on mesure les valeurs d'extinction de la masse réactionnelle en fonction du temps,
• on établit une courbe de réaction à partir des valeurs d'extinction mesurées en fonction du temps,
• on détermine la variation maximum d'extinction au moyen d'un procédé de régression et
• on détermine la concentration en fibrinogène à l'aide d'une courbe d'étalonnage, qui a été établie à partir d'une correspondance entre des concentrations connues en fibrinogène et des variations maximum d'extinction.

2. Procédé suivant la revendication 1, dans lequel le procédé de régression comprend les stades suivants :
• un premier stade dans lequel, par un premier intervalle de régression fixé à l'avance, on détermine une première variation maximum provisoire d'extinction de la courbe de réaction,
• dans un deuxième stade, dans lequel on détermine, à l'aide de la première variation maximum provisoire d'extinction qui a été déterminée et à l'aide de paramètres fixés à l'avance, un deuxième intervalle de régression,
• un troisième stade dans lequel on détermine, par le deuxième intervalle de régression, la variation maximum d'extinction.

3. Procédé suivant la revendication 1, dans lequel se procurer une masse réactionnelle contenant l'échantillon de plasma et de la thrombine comprend l'addition de thrombine à l'échantillon de plasma et dans lequel, entre l'instant de l'addition de la thrombine à l'échantillon de plasma et le début de la mesure des valeurs d'extinction de la masse réactionnelle, il s'écoule un laps de temps de 0,5 à 5 secondes, de préférence de 3 à 4 secondes.

4. Appareil (10) d'analyse automatique, comprenant au moins un dispositif (21, 27) de pipetage, un poste (30) de mesure photométrique ou néphélométrique et une unité de traitement de données, **caractérisé en ce que** l'appareil (10) d'analyse comprend, en outre, un dispositif de commande configuré de manière à commander un procédé de détermination de la concentration en fibrinogène d'un échantillon de plasma suivant la méthode de Clauss, le procédé comprenant les stades suivants :
• on se procure une masse réactionnelle contenant l'échantillon de plasma et de la thrombine,
• on mesure les valeurs d'extinction de la masse réactionnelle en fonction du temps,
• on établit une courbe de réaction à partir des valeurs d'extinction mesurées en fonction du temps,
• on détermine la variation maximum d'extinction au moyen d'un procédé de régression et
• on détermine la concentration en fibrinogène à l'aide d'une courbe d'étalonnage, qui a été établie à partir d'une correspondance entre des concentrations connues en fibrinogène et des variations maximum d'extinction.

5. Appareil (10) d'analyse automatique suivant la revendication 4, comprenant, en outre, une première position (22) de réception de récipients de réaction, prévue pour se procurer des masses réactionnelles, et une deuxième position (32) de réception de récipients de réaction, associée au poste (30) de mesure, et un système (10, 11) de transport d'un récipient de réaction de la première à la deuxième position de réception, le dispositif de commande étant configuré, en outre, de manière à commander, en outre, les stades suivants :
• dans une première position (22) de réception, mise à disposition d'un récipient de réaction contenant la masse réactionnelle par addition de thrombine à un échantillon de plasma se trouvant dans le récipient de réaction,
• transport du récipient de réaction de la première à la deuxième position (23) de réception et ensuite
• mesure des valeurs d'extinction de la masse réactionnelle en fonction du temps dans le poste (30) de mesure,
dans lequel, entre l'instant de l'addition de la thrombine à l'échantillon de plasma et le début de la mesure des valeurs d'extinction de la masse réactionnelle, il s'écoule un laps de temps de 0,5 à 5 secondes, de préférence de 3 à 4 secondes.
